# DEMANDE DE BREVET EUROPEEN

(11) **EP 4 810 012 A1**
(43) Date de publication de la demande: **23.09.2026**
(21) Numéro de dépôt: 26165144.2
(22) Date de dépôt: 16.03.2026
(51) Int. Cl.: A61K 8/92, A61K 8/06, A61K 8/73, A61K 8/9789, A61Q 19/00

(54) **COMPOSITION POUR CRÈME COSMÉTIQUE, PROCÉDÉ DE PRÉPARATION D'UNE CRÈME COSMÉTIQUE ET UTILISATION D'UNE TELLE COMPOSITION À DES FINS COSMÉTIQUES**

(30) Priorité: 20.03.2025 FR 2502850
(71) Demandeur: Szwed, Annabelle, 56000 Vannes (FR)
(72) Inventeur: Szwed, Annabelle, 56000 Vannes (FR)
(74) Mandataire: Eidelsberg, Olivier Nathan

(57) **Abrégé**

Composition, notamment pour crème cosmétique, comprenant une phase aqueuse et une phase huileuse d'origine naturelle, caractérisée en ce que la phase huileuse représente de 50 à 60 % du poids total de la composition la phase aqueuse étant dispersée sous forme de gouttelettes dans la phase huileuse continue, représentant de 40 à 50 % du poids total de la composition.

## Description

|La présente invention se rapporte aux crèmes cosmétiques.

La viscosité, la couleur, l'odeur et l'homogénéité de nombreuses crèmes cosmétiques varient au cours du temps et suivant la température et les conditions de luminosité.

De US2023/0380421, il est décrit une composition pour constituer une crème solaire et anti moustique, comportant une phase aqueuse qui représente 28% en poids de la composition totale.

De CN105030650B, il est décrit des compositions pour former des crèmes pour la peau, comportant une phase huileuse représentant environ 25% en poids de la composition et une phase aqueuse représentant environ 75% en poids de la composition.

Ces crèmes cosmétique de l'art antérieur manquent de stabilité et se détériorent rapidement.

La présente invention vise à surmonter les inconvénients de l'art antérieur en proposant les moyens d'obtenir, sans ajout de conservateur, une composition pour former une crème cosmétique ayant une grande stabilité au cours du temps.

Suivant un premier aspect, l'invention pallie ces inconvénients par une composition, notamment pour crème cosmétique, comprenant une phase aqueuse et une phase huileuse d'origine naturelle, caractérisée en ce que la phase huileuse représente de 50 à 60 % du poids total de la composition, la phase aqueuse étant dispersée sous forme de gouttelettes dans la phase huileuse continue, représentant de 40 à 50 % du poids total de la composition.

La crème cosmétique obtenue présente ainsi une excellente stabilité dans le temps, notamment lorsqu'il y est incorporé de la cire d'abeille dans la phase huileuse et/ou un agent gélifiant et/ou épaississant dans la phase aqueuse.

De préférence, la phase huileuse contient de la cire d'abeille à raison de 5 à 8% en poids de la phase huileuse, de préférence de 5,5% à 7,5%.

La cire d'abeille permet au refroidissement d'augmenter à la fois la viscosité du produit, mais aussi de créer une gêne stérique favorisant le maintien de la suspension des gouttelettes gélifiées. La cire d'abeille permet de stabiliser la phase dispersée dans la phase continue. Les phénomènes d'instabilités sont ainsi retardés. La stabilité de la crème est assurée également par l'équilibre entre les proportions d'ingrédients hydrophiles et lipophiles. Enfin l'émulsion eau dans l'huile de la crème suivant l'invention assure l'internalisation de la phase aqueuse (sensible à la contamination microbienne de type bactérienne, levure et moisissure) au sein de la phase lipophile protectrice.

De préférence, la phase huileuse contient de 0,4 à 2 % en poids de la composition d'un agent gélifiant et/ou épaississant.

Comme gomme pour former un agent gélifiant et/ou épaississant, on peut notamment utiliser des matériaux principalement dérivés de sources naturelles. À titre d'exemple non limitatif, ces gommes gélifiantes comprennent la gomme arabique, l'agar-agar, l'alginate, l'acide alginique, l'alginate d'ammonium, l'amylopectine, l'alginate de calcium, le carraghénane calcique, la carnitine, le carraghénane, la dextrine, la gélatine, la gomme gellane, la gomme de guar, le chlorure de guar hydroxypropyltrimonium, l'hectorite, l'acide hyaluronique, la silice hydratée, l'hydroxypropylchitosane, l'hydroxypropylguar, la gomme karaya, le varech, la gomme de caroube, la gomme natta, l'alginate de potassium, le carraghénane de potassium, l'alginate de propylène glycol, la gomme de sclérote, le carboxyméthyl dextrane de sodium, le carraghénane de sodium, la gomme adragante, la gomme xanthane, la gomme biosacharide et leurs mélanges.

Des exemples d'épaississants hydrosolubles comprennent les polymères naturels hydrosolubles, les polymères synthétiques hydrosolubles, les minéraux argileux et l'anhydride silicique. Parmi les exemples non limitatifs de polymères naturels hydrosolubles, on peut citer la gomme arabique, la gomme adragante, la gomme karaya, la gomme de guar, la gomme gellane, la gomme de tara, la gomme de caroube, la gomme de tamarin, l'alginate de sodium, l'ester de propylèneglycol d'acide alginique, le carraghénane, le farcelluran, l'agar-agar, 25 pectines à haut degré de méthoxylation, pectines à bas degré de méthoxylation, xanthine, chitosane, amidon (par exemple, amidon dérivé du maïs, de la patate douce, du blé, du riz, de la patate douce et du tapioca, amidon α, amidon soluble), polysaccharides de fermentation (notamment gomme de xanthane, pullulane, carcirane, dextrane), hétéropolysaccharides acides dérivés du cal de plantes appartenant au genre Polyantes sp. (par exemple, polysaccharide tubéreux), protéines (par exemple, caséine sodique, gélatine, albumine), sulfate de chondroïtine et acide hyaluronique.

On préfère utiliser comme agent gélifiant et/ou épaississant une gomme, tout particulièrement la gomme de xanthane.

De préférence, la composition est exempte de conservateur et d'agent tensioactif autres que la cire d'abeille et les gommes, notamment autres que la cire d'abeille et la gomme de xanthane.

On préfère ajouter un anti-oxydant comme un extrait de romarin ou de la vitamine E (par exemple tocophérol), à raison de 0,1 à 0,2% du poids total de la composition.

La phase huileuse de la composition peut comprendre des extraits de la famille des bétulacées, en particulier un extrait de noisette du genre corylus, et un extrait de la famille des punicacées, en particulier un extrait de graine de grenade du genre punica, à raison de 1,0 à 2,0% du poids total de la composition.

La phase huileuse peut contenir des huiles végétales comme de l'huile de tournesol ou de l'huile d'olive ou des macérats de fleurs ou de racines végétales de ces huiles.

Comme ingrédient hydrophile on peut utiliser des hydrolats de fleurs ou de feuilles de végétaux.

De préférence, suivant un deuxième aspect de l'invention, indépendant du premier aspect, mais pouvant de manière favorable être mis en œuvre en combinaison avec celui-ci, la phase aqueuse représente entre 35% et 45% du poids total de la composition, de préférence entre 35% et 42%, tandis que la phase huileuse représente entre 55% et 65% du poids total de la composition, de préférence entre 58% et 65%.

Lorsque la proportion en poids de la phase aqueuse dans la composition est inférieure ou égale à 42%, la composition a, de manière très avantageuse, une grande innocuité microbiologique.

Ainsi, suivant un mode de réalisation particulièrement stable et présentant une innocuité élevée, la proportion en poids de la phase aqueuse dans la composition est supérieure ou égale à 40% et inférieure ou égale à 45%, de préférence inférieure ou égale à 42%.

L'invention vise également un procédé de préparation d'une crème cosmétique, dans lequel on mélange les ingrédients d'une phase huileuse telle que définie ci-dessus sans agent gélifiant et/ou épaississant dans un mélangeur rotatif à 400 à 500 tours à la minute en chauffant entre 60 et 63°C jusqu'à une dissolution complète de la cire d'abeille, en parallèle on chauffe, sous agitation magnétique entre 57 et 63°C, les ingrédients d'une phase aqueuse telle que définie ci-dessus sans agent gélifiant et/ou épaississant, puis on ajoute un agent gélifiant et/ou épaississant, de préférence une gomme, notamment de la gomme de xanthane, pour former un gel, lorsque les deux phases sont à la même température à plus ou moins 3°C, on procède à l'émulsion eau dans huile des deux phases sous une agitation d'environ 1 000 tours à la minute, tout en conservant la température, puis on laisse refroidir et, éventuellement, on ajoute un actif et/ou de l'antioxydant.

La présente invention se rapporte également à l'utilisation d'une composition, comprenant une phase aqueuse et une phase huileuse d'origine naturelle, la phase huileuse représentant de 50 à 60 % en poids total de la composition et la phase aqueuse étant dispersée sous forme de gouttelettes dans la phase huileuse continue et représentant de 40 à 50 % en poids total de la composition à des fins cosmétiques.

Les exemples suivants illustrent l'invention.

Une émulsion est un mélange homogène de deux liquides ou phases, peu miscibles. Ce mélange correspond à une dispersion de gouttelettes de l'une des phases dans l'autre. Les émulsions décrites ci-dessous, à savoir : *Crème Détox, Crème Apaisante, Crème Régénérante, Crème Glow et Crème Protectrice,* sont des émulsions dites indirectes ou inverses (eau dans huile). Ce type d'émulsion présente une phase continue (externe) lipophile et une phase dispersée (interne) de nature hydrophile. Il s'agit d'émulsions de texture fluide.

La phase lipophile peut se composer de plusieurs types d'ingrédients, notamment la cire d'abeille, des huiles végétales, des macérât huileux, des extraits huileux ou analogues.

La phase hydrophile peut se composer de plusieurs types d'ingrédients, notamment des hydrolats et eaux florales, de la gomme de xanthane ou analogues.

Les étapes suivantes sont réalisées :

Peser les huiles, les macérâts végétaux et la cire d'abeille émiettée dans un bécher en verre préalablement désinfecté.

Procéder au mélange de la phase huileuse sous Turbotest à 350 rpm.

Chauffer jusqu'à la dissolution totale de la cire à 58-60°C.

En parallèle, peser les hydrolats un à un dans un bécher en verre préalablement désinfecté.

Chauffer sous agitation magnétique à vitesse moyenne jusqu'à atteindre une température comprise entre 55 et 60°C.

Procéder à la formation du gel par ajout de la gomme de xanthane en pluie.

Lorsque les deux phases atteignent la même température (à ±3°C), procéder à l'émulsion eau-dans-huile sous forte agitation jusqu'à l'obtention d'un vortex (environ 1000 rpm).

Poursuivre le chauffage pendant environ 10 minutes, puis abaisser la température de la plaque.

Laisser refroidir jusqu'à 40°C, puis ajouter l'actif et l'extrait de romarin. Homogénéiser, puis conditionner.

Les compositions des différents exemples sont données dans les tableaux ci-après, « %PF » signifiant « % en poids dans la composition finale »

**Tableau 1**

| Crème DETOX | %PF |
|---|---|
| Hydrolat de Géranium Bio | 20 |
| Hydrola de Bleuet BIO | 19 |
| Hydrolat de Ciste Ladanifère BIO | 7,3 |
| Gomme de Xanthane | 0,5 |
| Macérât de racines de Carotte BIO | 25,5 |
| "Huile végétale de Noyaux Cerise Bio | 20,5 |
| Nactiol N+ | 2 |
| Romarin extrait huileux | 0,2 |
| Cire d'abeille jaune Bio | 5 |
| TOTAL | 100 |

**Tableau 2**

| Crème apaisante | %PF |
|---|---|
| Hydrolat de Lavande fine Sauvage BIO | 8 |
| Hydrolat d'Helichryse Italienne | 3 |
| Hydrolat de Camomille romaine BIO | 35 |
| Gomme de Xanthane | 0,5 |
| Macérât Huileux de Calendula | 43,9 |
| Nactiol G+ | 2 |
| Huile de Chanvre BIO | 2 |
| Romarin extrait huileux | 0,1 |
| Cire d'abeille jaune | 5,5 |
| TOTAL | 100 |

**Tableau 3**

| Crème régénérante | % PF |
|---|---|
| Hydrolat de Laurier Noble | 10 |
| Hydrolat de géranium | 30 |
| Gomme de Xanthane | 0,5 |
| Huile d'amandon de pruneau | 2 |
| Huile de mirabelle | 2 |
| Huile végétale de pépins de raisin | 13 |
| Huile de graine de chia | 34,9 |
| Nactiol G+ | 2 |
| Romarin Extrait huileux | 0,1 |
| Cire d'abeille jaune | 5,5 |
| TOTAL | 100 |

**Tableau 4**

| Crème Glow | % PF |
|---|---|
| Hydrolat de menthe poivrée BIO | 14 |
| Hydrolat de cyprès BIO | 13 |
| Hydrolat de Pin Sylvestre BIO | 13 |
| Gomme de Xanthane | 0,5 |
| Huile de noisette BIO | 10 |
| Huile de pépins de pomme BIO | 8 |
| Huile de Carthame BIO | 8,9 |
| Huile d'Argousier BIO | 3 |
| Nactiol N+ | 2 |
| Macérât Huileux de Calendula | 20 |
| Extrait Huileux de Romarin | 0,1 |
| Cire d'Abeille | 7,5 |
| TOTAL | 100 |

Une émulsion reste stable tant que les deux phases qui la composent sont homogènes.

Cette stabilité est étudiée par évaluation des variations des différents paramètres physico-chimiques qui caractérise ledit produit. Certains facteurs extrinsèques peuvent être considérés, à savoir :
Choc thermique par cycle climatique : Variation des températures entre 4°C et 40°C toutes les 24h pendant 5 jours.

Étude du vieillissement accéléré jusqu'à 3 mois : à 4°C, 40°C et température ambiante.

Stimulation à la lumière naturelle.

Vérification en temps réel jusqu'à 3 ans avec intervalle de contrôle annuel.

Sont notamment étudiés, surveillés et contrôlés les évènements de déstabilisation, comme le crémage, la floculation, le murissement d'Ostwald ou l'inversion de phase.

Les émulsions inverses, comme présentées ici, peuvent reposer sur les répulsions stériques (phénomène physique) conférées par la cire d'abeille. La densité de la phase continue joue un rôle décisif contre la sédimentation. La viscosité de la phase interne, qui est ici importante grâce à l'importante concentration de gomme xanthane, va de plus avoir un impact sur la mobilité des gouttelettes et leur séparation.

Les études de stabilités microbiologiques reposent sur des dénombrements microbiens selon les normes NF EN ISO 21149 (bactéries aérobies mésophiles) et NF EN ISO 16212 (levures et moisissures) et de recherche de germes spécifiés selon les normes NF en ISO 18416 (*Candida Albicans*), NF EN ISO 21150 (*E. Coli*), NF EN ISO 22717 (*Pseudomonas Aeruginos*), NF EN ISO 22718 (*Staphylococcus Aureus*).

Ces analyses sont faites de manière continue sur 120 jours avec prélèvements réguliers sur la durée totale de l'analyse.

Afin de démontrer l'intérêt de la cire d'abeille dans la stabilisation de la formule, nous avons proposé une contre étude de stabilité sur une formule formant un exemple suivant l'invention, tel que défini au Tableau 5, et sur une formule d'exemple comparatif tel que défini au Tableau 6.

Conditions du test
- Réalisé sur une quantité de 250gr de produit final (6 échantillons de 30mL)
- Température ambiante de 17,7°C
- Chaque duplicata d'échantillon fut déposé à J+1 au réfrigérateur, à l'étuve et dans le laboratoire à température ambiante.

**Tableau 5**

| Crème protectrice | INCI | %PF | %MA |
|---|---|---|---|
| Hydrolat de menthe poivrée Bio | Mentha Piperita Leaf water | 35 | 100 |
| Hydrolat de Cyprès Bio | Cupressus sempervirens Leaf water | 5 | 100 |
| Gomme de Xanthane | Xanthane Gum | 0,45 | 100 |
| Huile de Carthame Bio | Carthamus tinctorius seed oil | 52,45 | 100 |
| Nactiol N+ | Corylus Avellana Seed Extract | 2 | 100 |
| Extrait huileux de Romarin | Helianthus Annuus Seed Oil Rosmanirus Officinalis Leaf Extract | 0,1 | 75 |
| | | | 25 |
| Cire d'abeille | Cera Alba | 5,0 | 100 |

Résultats:
- Choc thermique par cycle climatique + test microbiologique : CONFORME
- Étude du vieillissement accéléré jusqu'à 3 mois : CONFORME
- Stimulation à la lumière naturelle jusqu'à 3 mois : CONFORME.

**Tableau 6**

| | INCI | %PF | %MA |
|---|---|---|---|
| Hydrolat de menthe poivrée Bio | Mentha Piperita Leaf water | 35 | 100 |
| Hydrolat de Cyprès Bio | Cupressus sempervirens Leaf water | 5 | 100 |
| Gomme de Xanthane | Xanthane Gum | 0,45 | 100 |
| Huile de Carthame Bio | Carthamus tinctorius seed oil | 55,45 | 100 |
| Nactiol N+ | Corylus Avellana Seed Extract | 2 | 100 |
| Extrait huileux de Romarin | Helianthus Annuus Seed Oil Rosmanirus Officinalis Leaf Extract | 0,1 | 75 |
| | | | 25 |
| Cire d'abeille | Cera Alba | 2,0 | 100 |

Résultats :
- Choc thermique par cycle climatique + test microbiologique : NON-CONFORME
- Étude du vieillissement accéléré jusqu'à 3 mois : NON-CONFORME

La crème ne contenant que 2% de cire d'abeille (qsp phase huileuse) présente un phénomène d'instabilité visible dès la première semaine d'étude. En effet, un relargage d'huile peut être observé à différentes températures (dans l'ordre : Température ambiante > 4°C > 40°C).

Un produit cosmétique est une substance ou un mélange destiné à être mis en contact avec les parties superficielles du corps humain tels que l'épiderme, les systèmes pileux et capillaires, les ongles, les lèvres et les organes génitaux externes, les dents et les muqueuses buccales, en vue, exclusivement ou principalement, de les nettoyer, parfumer, d'en modifier l'aspect, de les protéger, de les maintenir en bon état ou de corriger les odeurs corporelles.

D'autre part, les crèmes respectivement dite régénérante du Tableau 3, dite apaisante du Tableau 2 et Détox du Tableau 1 ont fait l'objet d'analyses consistant à dénombrer des bactéries aérobies mésophiles suivant la Norme NF EN ISO 21149, à dénombrer des levures et moisissures suivant la Norme NF EN ISO 16212, à détecter des germes spécifiés et non spécifiés suivant la norme NF EN ISO 18415, à détecter *Candida albicans suivant la norme* NF EN ISO 18416, à détecter *Escherichia coli suivant la norme* NF EN ISO 21150, à détecter *Pseudomonas aeruginosa suivant la norme* NF EN ISO 22717 et à détecter *Staphylococcus aureus suivant la norme* NF EN ISO 22718.

Les échantillons suivants ont été analysés :

| | |
|---|---|
| 1 | Crème apaisante - Date de fabrication : T0 - 3 jours |
| | Conditionnement : 2x Flacons en verre transparent avec poche de 50 mL (LABLABO) et capuchon transparent |
| 2 | Crème apaisante - Date de fabrication = T0 - 3 jours |
| | Conditionnement : 2x Flacons Airless blanc opaque de 50mL (LABLABO) en PP |
| 3 | Crème apaisante - Date de fabrication = T0 - 3 jours |
| | Conditionnement : 2x Flacons en verre transparent avec poche de 50 mL (LABLABO) et capuchon transparent |
| 4 | Crème apaisante - Date de fabrication = T0 - 3 jours |
| | Conditionnement : 2x Flacons dits Airless blanc opaque de 50mL (LABLABO) en PP |
| 5 | Crème apaisante - Date de fabrication = T0 - 3 jours |
| | Conditionnement : 2x Flacons en verre transparent avec poche de 50 mL (LABLABO) et capuchon transparent |
| 6 | Crème apaisante - Date de fabrication = T0 - 3 jours |
| | Conditionnement : 2x Flacons dits Airless blanc opaque de 50mL (LABLABO) en PP |
| 28 | Crème régénérante - Date de fabrication : T0 - 3 jours |
| | Conditionnement : 2x Flacons en verre transparent avec poche de 50 mL (LABLABO) et capuchon transparent |
| 29 | Crème régénérante - Date de fabrication = T0 - 3 jours |
| | Conditionnement : 2x Flacons Airless blanc opaque de 50mL (LABLABO) en PP |
| 30 | Crème régénérante - Date de fabrication = T0 - 3 jours |
| | Conditionnement : 2x Flacons en verre transparent avec poche de 50 mL (LABLABO) et capuchon transparent |
| 31 | Crème régénérante - Date de fabrication = T0 - 3 jours |
| | Conditionnement : 2x Flacons dits Airless blanc opaque de 50mL (LABLABO) en PP |
| 32 | Crème régénérante - Date de fabrication = T0 - 3 jours |
| | Conditionnement : 2x Flacons en verre transparent avec poche de 50 mL (LABLABO) et capuchon transparent |
| 33 | Crème régénérante - Date de fabrication = T0 - 3 jours |
| | Conditionnement : 2x Flacons dits Airless blanc opaque de 50mL (LABLABO) en PP |
| 40 | Crème détox - Date de fabrication : T0 - 3 jours |
| | Conditionnement : 2x Flacons en verre transparent avec poche de 50 mL (LABLABO) et capuchon transparent |
| 41 | Crème détox - Date de fabrication = T0 - 3 jours |
| | Conditionnement : 2x Flacons Airless blanc opaque de 50mL (LABLABO) en PP |
| 42 | Crème détox - Date de fabrication = T0 - 3 jours |
| | Conditionnement : 2x Flacons en verre transparent avec poche de 50 mL (LABLABO) et capuchon transparent |
| 43 | Crème détox - Date de fabrication = T0 - 3 jours |
| | Conditionnement : 2x Flacons dits Airless blanc opaque de 50mL (LABLABO) en PP |
| 44 | Crème détox - Date de fabrication = T0 - 3 jours |
| | Conditionnement : 2x Flacons en verre transparent avec poche de 50 mL (LABLABO) et capuchon transparent |
| 45 | Crème détox - Date de fabrication = T0 - 3 jours |
| | Conditionnement : 2x Flacons dits Airless blanc opaque de 50mL (LABLABO) en PP |

Les échantillons ont été conservés 120 jours à température ambiante à l'abri de la lumière.

Le protocole d'essai a été le suivant :
- Pesée : 1 g du produit
- Diluant : Bouillon EUGON LT 100

Les analyses ont été réalisées dans le temps suivant les dates mentionnées dans le tableau ci-dessous.

| Analyses réalisées | Date de prélèvement |
|---|---|
| DGAT | T0 |
| DLMT | T3 = T0 + 3 jours |
| Germes spécifiés et non spécifiés (SPEC) | T6 = T0 + 6 jours |
| | T13 = T0 + 13 jours |
| *Staphylococcus aureus* | T20 = T0 + 20 jours |
| *Pseudomonas aeruginosa Escherichia coli* | T30 = T0 + 30 jours |
| | T45 = T0 + 45 jours |
| *Candida albicans* | T60 = T0 + 60 jours |
| | T90 = T0 + 90 jours |
| | T120 = T0 + 120 jours |

A la vue des résultats, les analyses ont été arrêtées à T13 pour la crème apaisante et à T20 pour la crème détox.

Les résultats suivants ont été obtenus.

Résultats des dénombrements de la flore aérobie mésophile (DGAT et des levures et moisissures (DLMT) sur le produit 28 :

| Date analyse | DGAT UFC / g | DLMT UFC / g |
|---|---|---|
| T0 | <10 | <10 moisissures, <10 levures |
| T3 | <10 | <10 moisissures, <10 levures |
| T6 | <10 | <10 moisissures, <10 levures |
| T13 | <10 | <10 moisissures, <10 levures |
| T20 | <10 | <10 moisissures, <10 levures |
| T30 | <10 | <10 moisissures, <10 levures |
| T45 | <10 | <10 moisissures, <10 levures |
| T60 | <10 | <10 moisissures, <10 levures |
| T90 | <10 | <10 moisissures, <10 levures |
| T120 | <10 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 28 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté / g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté |
| T13 | Non détecté | Non détecté | Non détecté | Non détecté | Non détecté |
| T20 | Non détecté | Non détecté | Non détecté | Non détecté | Non détecté |
| T30 | Non détecté | Non détecté | Non détecté | Non détecté | Non détecté |
| T45 | Non détecté | Non détecté | Non détecté | Non détecté | Non détecté |
| T60 | Non détecté | Non détecté | Non détecté | Non détecté | Non détecté |
| T90 | Non détecté | Non détecté | Non détecté | Non détecté | Non détecté |
| T120 | Non détecté | Non détecté | Non détecté | Non détecté | Non détecté |

Résultats des dénombrements de la flore aérobie mésophile (DGAT et des levures et moisissures (DLMT) sur le produit 29 :

| Date analyse | DGAT UFC / g | DLMT UFC / g |
|---|---|---|
| T0 | <10 | <10 moisissures, <10 levures |
| T3 | <10 | <10 moisissures, <10 levures |
| T6 | <10 | <10 moisissures, <10 levures |
| T13 | <10 | <10 moisissures, <10 levures |
| T20 | <10 | <10 moisissures, <10 levures |
| T30 | <10 | <10 moisissures, <10 levures |
| T45 | <10 | <10 moisissures, <10 levures |
| T60 | <10 | <10 moisissures, <10 levures |
| T90 | <10 | <10 moisissures, <10 levures |
| T120 | <10 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 29 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté / g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté |
| T13 | Non détecté | Non détecté | Non détecté | Non détecté | Non détecté |
| T20 | Non détecté | Non détecté | Non détecté | Non détecté | Non détecté |
| T30 | Non détecté | Non détecté | Non détecté | Non détecté | Non détecté |
| T45 | Non détecté | Non détecté | Non détecté | Non détecté | Non détecté |
| T60 | Non détecté | Non détecté | Non détecté | Non détecté | Non détecté |
| T90 | Non détecté | Non détecté | Non détecté | Non détecté | Non détecté |
| T120 | Non détecté | Non détecté | Non détecté | Non détecté | Non détecté |

Résultats des dénombrements de la flore aérobie mésophile (DGAT et des levures et moisissures (DLMT) sur le produit 30 :

| Date analyse | DGAT UFC / g | DLMT UFC / g |
|---|---|---|
| T0 | <10 | <10 moisissures, <10 levures |
| T3 | <10 | <10 moisissures, <10 levures |
| T6 | <10 | <10 moisissures, <10 levures |
| T13 | <10 | <10 moisissures, <10 levures |
| T20 | <10 | <10 moisissures, <10 levures |
| T30 | <10 | <10 moisissures, <10 levures |
| T45 | <10 | <10 moisissures, <10 levures |
| T60 | <10 | <10 moisissures, <10 levures |
| T90 | <10 | <10 moisissures, <10 levures |
| T120 | <10 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 30 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté / g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté |
| T13 | Non détecté | Non détecté | Non détecté | Non détecté | Non détecté |
| T20 | Non détecté | Non détecté | Non détecté | Non détecté | Non détecté |
| T30 | Non détecté | Non détecté | Non détecté | Non détecté | Non détecté |
| T45 | Non détecté | Non détecté | Non détecté | Non détecté | Non détecté |
| T60 | Non détecté | Non détecté | Non détecté | Non détecté | Non détecté |
| T90 | Non détecté | Non détecté | Non détecté | Non détecté | Non détecté |
| T120 | Non détecté | Non détecté | Non détecté | Non détecté | Non détecté |

Résultats des dénombrements de la flore aérobie mésophile (DGAT) et des levures et moisissures (DLMT) sur le produit 31 :

| Date analyse | DGAT UFC / g | DLMT UFC / g |
|---|---|---|
| T0 | <10 | <10 moisissures, <10 levures |
| T3 | <10 | <10 moisissures, <10 levures |
| T6 | <10 | <10 moisissures, <10 levures |
| T13 | <10 | <10 moisissures, <10 levures |
| T20 | <10 | <10 moisissures, <10 levures |
| T30 | <10 | <10 moisissures, <10 levures |
| T45 | <10 | <10 moisissures, <10 levures |
| T60 | <10 | <10 moisissures, <10 levures |
| T90 | <10 | <10 moisissures, <10 levures |
| T120 | <10 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 31 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté / g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté |
| T13 | Non détecté | Non détecté | Non détecté | Non détecté | Non détecté |
| T20 | Non détecté | Non détecté | Non détecté | Non détecté | Non détecté |
| T30 | Non détecté | Non détecté | Non détecté | Non détecté | Non détecté |
| T45 | Non détecté | Non détecté | Non détecté | Non détecté | Non détecté |
| T60 | Non détecté | Non détecté | Non détecté | Non détecté | Non détecté |
| T90 | Non détecté | Non détecté | Non détecté | Non détecté | Non détecté |
| T120 | Non détecté | Non détecté | Non détecté | Non détecté | Non détecté |

Résultats des dénombrements de la flore aérobie mésophile (DGAT) et des levures et moisissures (DLMT) sur le produit 32 :

| Date analyse | DGAT UFC / g | DLMT UFC / g |
|---|---|---|
| T0 | <10 | <10 moisissures, <10 levures |
| T3 | <10 | <10 moisissures, <10 levures |
| T6 | <10 | <10 moisissures, <10 levures |
| T13 | <10 | <10 moisissures, <10 levures |
| T20 | <10 | <10 moisissures, <10 levures |
| T30 | <10 | <10 moisissures, <10 levures |
| T45 | <10 | <10 moisissures, <10 levures |
| T60 | <10 | <10 moisissures, <10 levures |
| T90 | <10 | <10 moisissures, <10 levures |
| T120 | <10 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 32 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté / g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté |
| T13 | Non détecté | Non détecté | Non détecté | Non détecté | Non détecté |
| T20 | Non détecté | Non détecté | Non détecté | Non détecté | Non détecté |
| T30 | Non détecté | Non détecté | Non détecté | Non détecté | Non détecté |
| T45 | Non détecté | Non détecté | Non détecté | Non détecté | Non détecté |
| T60 | Non détecté | Non détecté | Non détecté | Non détecté | Non détecté |
| T90 | Non détecté | Non détecté | Non détecté | Non détecté | Non détecté |
| T120 | Non détecté | Non détecté | Non détecté | Non détecté | Non détecté |

Résultats des dénombrements de la flore aérobie mésophile (DGAT) et des levures et moisissures (DLMT) sur le produit 33 :

| Date analyse | DGAT UFC / g | DLMT UFC / g |
|---|---|---|
| T0 | <10 | <10 moisissures, <10 levures |
| T3 | <10 | <10 moisissures, <10 levures |
| T6 | <10 | <10 moisissures, <10 levures |
| T13 | <10 | <10 moisissures, <10 levures |
| T20 | <10 | <10 moisissures, <10 levures |
| T30 | <10 | <10 moisissures, <10 levures |
| T45 | <10 | <10 moisissures, <10 levures |
| T60 | <10 | <10 moisissures, <10 levures |
| T90 | <10 | <10 moisissures, <10 levures |
| T120 | <10 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 33 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté / g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté |
| T13 | Non détecté | Non détecté | Non détecté | Non détecté | Non détecté |
| T20 | Non détecté | Non détecté | Non détecté | Non détecté | Non détecté |
| T30 | Non détecté | Non détecté | Non détecté | Non détecté | Non détecté |
| T45 | Non détecté | Non détecté | Non détecté | Non détecté | Non détecté |
| T60 | Non détecté | Non détecté | Non détecté | Non détecté | Non détecté |
| T90 | Non détecté | Non détecté | Non détecté | Non détecté | Non détecté |
| T120 | Non détecté | Non détecté | Non détecté | Non détecté | Non détecté |

Résultats des dénombrements de la flore aérobie mésophile (DGAT et des levures et moisissures (DLMT) sur le produit 1 :

| Date analyse | DGAT UFC / g | DLMT UFC / g |
|---|---|---|
| T0 | 130 | <10 moisissures, <10 levures |
| T3 | 160 | <10 moisissures, <10 levures |
| T6 | 110 | 210 moisissures, <10 levures |
| T13 | 100 | 700 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 1 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | | | | |
| | | Détecté / Non détecté /g | Détecté / Non détecté / g | Détecté / Non détecté / g | Détecté / Non détecté / g |
| T0 | Non détecté | Non détecté | Non détecté | Non détecté | Non détecté |
| T3 | Non détecté | Non détecté | Non détecté | Non détecté | Non détecté |
| T6 | Non détecté | Non détecté | Non détecté | Non détecté | Non détecté (présence de moisissures) |
| T13 | Non détecté | Non détecté | Non détecté | Non détecté | Non détecté (présence de moisissures) |

Résultats des dénombrements de la flore aérobie mésophile (DGAT et des levures et moisissures (DLMT) sur le produit 2 :

| Date analyse | DGAT UFC / g | DLMT UFC / g |
|---|---|---|
| T0 | 140 | 480 moisissures, <10 levures |
| T3 | 80 | 40 moisissures, <10 levures |
| T6 | 60 | 40 moisissures, <10 levures |
| T13 | 50 | 850 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 2 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté (présence de moisissures) |
| T3 | Non détecté (présence de bacilles gram + sporulés) | Non détecté | Non détecté | Non détecté | Non détecté (présence de moisissures) |
| T6 | Non détecté (présence de bacilles gram + sporulés) | Non détecté | Non détecté | Non détecté | Non détecté (présence de moisissures) |
| T13 | Non détecté (présence de bacilles gram + sporulés) | Non détecté | Non détecté | Non détecté | Non détecté (présence de moisissures) |

Résultats des dénombrements de la flore aérobie mésophile (DGAT et des levures et moisissures (DLMT) sur le produit 3 :

| Date analyse | DGAT UFC / g | DLMT UFC / g |
|---|---|---|
| T0 | 210 | 620 moisissures, <10 levures |
| T3 | 140 | 30 moisissures, <10 levures |
| T6 | 50 | <10 moisissures, <10 levures |
| T13 | 60 | 120 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 3 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté | Non détecté | Non détecté | Non détecté | Non détecté (présence de moisissures) |
| T3 | Non détecté | Non détecté | Non détecté | Non détecté | Non détecté (présence de moisissures) |
| T6 | Non détecté | Non détecté | Non détecté | Non détecté | Non détecté (présence de moisissures) |
| T13 | Non détecté | Non détecté | Non détecté | Non détecté | Non détecté (présence de moisissures) |

Résultats des dénombrements de la flore aérobie mésophile (DGAT) et des levures et moisissures (DLMT) sur le produit 4 :

| Date analyse | DGAT UFC / g | DLMT UFC / g |
|---|---|---|
| T0 | 400 | <10 moisissures, <10 levures |
| T3 | 10 | <10 moisissures, <10 levures |
| T6 | 60 | <10 moisissures, <10 levures |
| T13 | 150 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 4 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté |

Résultats des dénombrements de la flore aérobie mésophile (DGAT) et des levures et moisissures (DLMT) sur le produit 5 :

| Date analyse | DGAT UFC / g | DLMT UFC / g |
|---|---|---|
| T0 | 140 | <10 moisissures, <10 levures |
| T3 | 110 | <10 moisissures, <10 levures |
| T6 | 120 | <10 moisissures, <10 levures |
| T13 | 140 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 5 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté |

Résultats des dénombrements de la flore aérobie mésophile (DGAT) et des levures et moisissures (DLMT) sur le produit 6 :

| Date analyse | DGAT UFC / g | DLMT UFC / g |
|---|---|---|
| T0 | 30 | <10 moisissures, <10 levures |
| T3 | 50 | <10 moisissures, <10 levures |
| T6 | 30 | 490 moisissures, <10 levures |
| T13 | 30 | 1800 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 6 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté (présence de moisissures) |
| T13 | Non détecté (présence de bacilles gram + sporulés) | Non détecté | Non détecté | Non détecté | Non détecté (présence de moisissures) |

Résultats des dénombrements de la flore aérobie mésophile (DGAT et des levures et moisissures (DLMT) sur le produit 40 :

| Date analyse | DGAT UFC / g | DLMT UFC / g |
|---|---|---|
| T0 | 50 | <10 moisissures, <10 levures |
| T3 | 20 | <10 moisissures, <10 levures |
| T6 | 120 | <10 moisissures, <10 levures |
| T13 | 40 | 30 moisissures, <10 levures |
| T20 | 20 | 6600 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 40 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté (présence de moisissures) |
| T20 | Non détecté (présence de bacilles gram + sporulés) | Non détecté | Non détecté | Non détecté | Non détecté (présence de moisissures) |

Résultats des dénombrements de la flore aérobie mésophile (DGAT et des levures et moisissures (DLMT) sur le produit 41 :

| Date analyse | DGAT UFC / g | DLMT UFC / g |
|---|---|---|
| T0 | 30 | <10 moisissures, <10 levures |
| T3 | 20 | <10 moisissures, <10 levures |
| T6 | 30 | <10 moisissures, <10 levures |
| T13 | 40 | 10 moisissures, <10 levures |
| T20 | 40 | 1200 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 41 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté /g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté (présence de moisissures) |
| T20 | Non détecté (présence de bacilles gram + sporulés) | Non détecté | Non détecté | Non détecté | Non détecté (présence de moisissures) |

Résultats des dénombrements de la flore aérobie mésophile (DGAT et des levures et moisissures (DLMT) sur le produit 42 :

| Date analyse | DGAT UFC / g | DLMT UFC / g |
|---|---|---|
| T0 | 40 | <10 moisissures, <10 levures |
| T3 | 10 | <10 moisissures, <10 levures |
| T6 | 10 | <10 moisissures, <10 levures |
| T13 | 30 | 1400 moisissures, <10 levures |
| T20 | 20 | 3400 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 42 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté /g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté (présence de moisissures) |
| T20 | Non détecté (présence de bacilles gram + sporulés) | Non détecté | Non détecté | Non détecté | Non détecté (présence de moisissures) |

Résultats des dénombrements de la flore aérobie mésophile (DGAT) et des levures et moisissures (DLMT) sur le produit 43 :

| Date analyse | DGAT UFC / g | DLMT UFC / g |
|---|---|---|
| T0 | 30 | <10 moisissures, <10 levures |
| T3 | 30 | <10 moisissures, <10 levures |
| T6 | 30 | <10 moisissures, <10 levures |
| T13 | 70 | <10 moisissures, <10 levures |
| T20 | 30 | <10 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 43 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté /g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté |
| T20 | Non détecté (présence de bacilles gram + sporulés) | Non détecté | Non détecté | Non détecté | Non détecté |

Résultats des dénombrements de la flore aérobie mésophile (DGAT) et des levures et moisissures (DLMT) sur le produit 44 :

| Date analyse | DGAT UFC / g | DLMT UFC / g |
|---|---|---|
| T0 | 30 | <10 moisissures, <10 levures |
| T3 | 30 | <10 moisissures, <10 levures |
| T6 | 40 | <10 moisissures, <10 levures |
| T13 | 10 | 4200 moisissures, <10 levures |
| T20 | 20 | 6300 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 44 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté (présence de moisissures) |
| T20 | Non détecté (présence de bacilles gram + sporulés) | Non détecté | Non détecté | Non détecté | Non détecté (présence de moisissures) |

Résultats des dénombrements de la flore aérobie mésophile (DGAT) et des levures et moisissures (DLMT) sur le produit 45 :

| Date analyse | DGAT UFC / g | DLMT UFC / g |
|---|---|---|
| T0 | <10 | <10 moisissures, <10 levures |
| T3 | 30 | <10 moisissures, <10 levures |
| T6 | 10 | <10 moisissures, <10 levures |
| T13 | 10 | 30 moisissures, <10 levures |
| T20 | 10 | 6600 moisissures, <10 levures |

Résultats des détections des microorganismes spécifiés et non spécifiés, détection *Staphylococcus aureus,* de *Pseudomonas aeruginosa, d'Escherichia coli* et de *Candida albicans* dans le produit 45 :

| Date analyse | Germes Spécifiés | Détection *Staphylococcus aureus* | Détection Pseudomonas aeruginosa | Détection *Escherichia coli* | Détection *Candida albicans* |
|---|---|---|---|---|---|
| | Détecté / Non détecté / g | Détecté / Non détecté / g | Détecté / Non détecté / g | Détecté / Non détecté / g | Détecté / Non détecté / g |
| T0 | Non détecté (présence de bacilles gram + sporulés) | Non détecté | Non détecté | Non détecté | Non détecté |
| T3 | Non détecté (présence de bacilles gram + sporulés) | Non détecté | Non détecté | Non détecté | Non détecté |
| T6 | Non détecté (présence de bacilles gram + sporulés) | Non détecté | Non détecté | Non détecté | Non détecté |
| T13 | Non détecté (présence de bacilles gram + sporulés) | Non détecté | Non détecté | Non détecté | Non détecté (présence de moisissures) |
| T20 | Non détecté (présence de bacilles gram + sporulés) | Non détecté | Non détecté | Non détecté | Non détecté (présence de moisissures) |

## Revendications

1. Utilisation à des fins cosmétiques d'une composition comprenant une phase aqueuse et une phase huileuse d'origine naturelle, la phase huileuse représentant de 50 à 60 % du poids total de la composition et la phase aqueuse étant dispersée sous forme de gouttelettes dans la phase huileuse continue et représentant de 40 à 50 % du poids total de la composition.

2. Utilisation à des fins cosmétiques d'une composition comprenant une phase aqueuse et une phase huileuse d'origine naturelle, la phase huileuse représentant de 55 à 65 % du poids total de la composition et la phase aqueuse étant dispersée sous forme de gouttelettes dans la phase huileuse continue et représentant de 35 à 45 % du poids total de la composition.

3. Utilisation suivant la revendication 2, **caractérisée en ce que** la phase huileuse représentant de 58 à 65 % du poids total de la composition et la phase aqueuse étant dispersée sous forme de gouttelettes dans la phase huileuse continue et représentant de 35 à 42 % du poids total de la composition.

4. Utilisation suivant la revendication 1, 2 ou 3, **caractérisée en ce que** la phase huileuse contient de la cire d'abeille à raison de 5 à 8% en poids de la phase huileuse, de préférence de 5,5% à 7,5%.

5. Utilisation suivant l'une des revendications 1 à 4, **caractérisée en ce que** la phase aqueuse contient de 0,4 à 2 % en poids de la composition d'un agent gélifiant et/ou épaississant.

6. Utilisation suivant la revendication 5, **caractérisée en ce que** la composition comporte de la gomme de xanthane comme agent gélifiant, la gomme de xanthane représentant de 0,4 à 2 % du poids total de la composition.

7. Utilisation suivant l'une des revendications 1 à 6, **caractérisée en ce que** la phase huileuse de la composition comprend un antioxydant, comme un extrait de romarin ou de la vitamine E.

8. Utilisation suivant l'une des revendications précédentes, **caractérisée en ce que** la phase huileuse de la composition comprend, comme actif, des extraits de la famille des bétulacées ou de la famille des punicaceae.

9. Utilisation suivant l'une des revendications précédentes, **caractérisée en ce que** la phase huileuse contient des huiles végétales comme de l'huile de tournesol ou de l'huile de l'olive, ou des macérats de fleurs ou de racines végétales de ces huiles.

10. Utilisation suivant l'une des revendications précédentes, **caractérisée en ce que** la crème est exempte de conservateur et d'agent tensioactif autres que la cire d'abeille et les gommes, notamment autres que la cire d'abeille et la gomme de xanthane.

11. Composition, notamment pour crème cosmétique, comprenant une phase aqueuse et une phase huileuse d'origine naturelle, **caractérisée en ce que** la phase huileuse représente de 50 à 60 % du poids total de la composition la phase aqueuse étant dispersée sous forme de gouttelettes dans la phase huileuse continue, représentant de 40 à 50 % du poids total de la composition.

12. Composition, notamment pour crème cosmétique, comprenant une phase aqueuse et une phase huileuse d'origine naturelle, **caractérisée en ce que** la phase huileuse représente de 55 à 65 % du poids total de la composition, de préférence de 58% à 65%, la phase aqueuse étant dispersée sous forme de gouttelettes dans la phase huileuse continue, représentant de 35 à 45 % du poids total de la composition, de préférence de 35% à 42%.

13. Composition suivant la revendication 10, 11 ou 11, **caractérisée en ce que** la phase huileuse contient de la cire d'abeille à raison de 5 à 8% en poids de la phase huileuse, de préférence de 5,5% à 7,5%.

14. Composition suivant la revendication 11 ou 12, **caractérisée en ce que** la phase aqueuse contient de 0,4 à 2 % en poids de la composition d'un agent gélifiant et/ou épaississant.

15. Composition suivant la revendication 14, **caractérisée en ce que** la composition comporte de la gomme de xanthane comme agent gélifiant, la gomme de xanthane représentant de 0,4 à 2 % du poids total de la composition. |
